(19) Europäisches Patentamt
European Patent Office
Office européen des brevets

(11) **EP 4 541 197 A1**

(12) **EUROPEAN PATENT APPLICATION**

(43) Date of publication:
23.04.2025 Bulletin 2025/17

(21) Application number: 23204842.1

(22) Date of filing: 20.10.2023

(51) International Patent Classification (IPC):
*A22B 5/00* (2006.01)       *A22B 5/20* (2006.01)
*G01N 33/12* (2006.01)      *G06T 7/00* (2017.01)
*G06V 10/00* (2022.01)

(52) Cooperative Patent Classification (CPC):
A22B 5/201; A22B 5/0041; G01N 33/12;
G06V 10/774; G06V 10/82; G06V 20/64

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: IHFood A/S
2200 Copenhagen N (DK)

(72) Inventor: Sølvhøj, Jonas
2200 Copenhagen N (DK)

(74) Representative: Plougmann Vingtoft a/s
Strandvejen 70
2900 Hellerup (DK)

(54) **A SYSTEM AND A COMPUTER IMPLEMENTED METHOD OF REMOVING, AT LEAST PARTLY, A SPINAL COLUMN**

(57) The present invention relates to a system and a computer implemented method of removing, at least partly, a spinal column from a specific half carcass of a slaughtered animal of a particular species, said specific half carcass has been provided by a cut substantially along the median surface of said slaughtered animal thereby providing a median cut surface of said specific half-carcass. The invention makes use of trained neural network(s).

Fig. 1

## Description

The present invention relates to a system and a computer implemented method of removing, at least partly, a spinal column from a specific half carcass of a slaughtered animal of a particular species, said specific half carcass has been provided by a cut substantially along the median surface of said slaughtered animal thereby providing a median cut surface of said specific half-carcass. The invention makes use of trained neural network(s).

## BACKGROUND OF THE INVENTION

US 8,915,773 discloses a method for separation of the spinal column from a carcass middle part. The method disclosed therein comprises the steps of determining a cutting path for a cutting device for the separation of the spinal column from the carcass middle part; separating the spinal column from the carcass middle part by causing a relative movement between the middle part and the cutting device and simultaneously causing the cutting device to engage the carcass middle. The method includes: optically scanning the middle part to provide a scan of an outer surface of the carcass middle part and identifying and locating the spinal canal in the spinal column on the basis of digital processing. The cutting path is subsequently to the scanning determined on the basis of the position of the spinal canal and the cutting is carried subsequent to the scanning and determination of the cutting path.

The method disclosed in US 8,915,773 requires that the spinal cord is optically detectable and the accuracy of the method therefore depends on the accuracy with which the carcass has been split along the median surface and reveals the spinal canal. Thus, if the carcass is not split perfectly along the median surface, e.g. if the cut is laterally offset, the spinal canal may not be detectable in an optical scan of the half-carcass. When this occurs, the method disclosed in US 8,915,773 fails to provide cutting path and removal of the spinal column is to be removed by other means such as manually resulting in increased labour cost. In addition, the split may typically be offset typically up till 10 mm, or even up till 20 mm, from a perfect split and/or it may further be angled relatively the median surface. Thus, even if the spinal column is detectable, the use of the position of the spinal column may lead to a relative high offset of the cutting surface may be offset too much or too little resulting in that either too much meat or too little bone is removed.

In addition, the use of an offset may provide useful results when half-carcasses of similar sizes are considered. However, in many practical situations, half-carcasses comes in a variety of difference sizes and use of an offset as in US 8,915,773 may result for smaller half-carcasses in that valuable meat is cut away.

Hence, an improved method for removing the spinal column would be advantageous, and in particular a more efficient and/or reliable method for removing the spinal column would be advantageous.

## OBJECT OF THE INVENTION

It is an object of the invention to provide a cutting method not relying on identification of the spinal canal.
It is a further object of the invention to provide a cutting method substantially independent of the cutting path used to split a carcass into two half-carcasses.
It is a further object of the present invention to provide an alternative to the prior art.
In particular, it may be seen as an object of the present invention to provide a method and device that solves the above mentioned problems of the prior art.

## SUMMARY OF THE INVENTION

In a first aspect the invention relates to a computer implemented method of removing, at least partly, a spinal column from a specific half carcass of a slaughtered animal of a particular species, said specific half carcass has been provided by a cut substantially along the median surface of said slaughtered animal thereby providing a median cut surface of said specific half-carcass, the method comprising

- provide a first training set comprising

  ○ a plurality of digital images of different half carcasses of said particular species, wherein each of said digital images comprising an individual ground truth cut curve representing a projected or executed cut curve, preferably located in said median cut surface;

- provide a first trained neural network, said first trained neural network being computer implemented and trained to determine an actual cut curve in a digital image of a half carcass of said particular species not forming part of said first training set, wherein training of said first trained neural network is carried out on said first training set with use of said ground truth cut curves,
- determining a cut path by

  ○ provide at least one, such as one or more, specific digital image of said specific half carcass to be cut,
  ○ determine in said specific digital image(s) an actual cut curve for said half-carcass to be cut by use of the first trained neural network,
  ○ provide an actual cut angle progression representing a cut angle progression relatively to a pre-defined surface, such as the median surface, in a lengthwise direction of the half carcass, or a further actual cut curve,
  ○ combining said actual cut curve and said actual cut angle progression into said cut path (13), so that said cut path proceeds along said actual cut curve with an angle equal to said provided actual cut angle progression, or combining said actual cut curve and said further actual cut curve into said cut path as a path as the path going through said actual cut curve and said further actual cut curve,

- instructing a cutting device to cut the specific half carcass longitudinally along said provided cut path.

Terms used herein are used in a manner being ordinary to a skilled person. Some the used terms are elucidated here below:

*Removing, at least partly, a spinal column* refers to removing at least a part of the spinal column left over in the half carcass.

*Digital image* refers to an image comprising pixels (2D) or voxels (3D). A digital image is preferably taken from the ventral side of the half carcass. A digital image may in preferred embodiments, be two or more digital images of the same half carcass taken at different viewing angles. In embodiments where the digital image comprising two or more digital images, the digital image of a training set will be the two or more digital images.

*Individual ground truth cut curve (GTCc)* refers to that the ground truth cut curve is established individually for each digital image. By this, the ground truth is valid for an individual image. However, if two or more images are highly alike, similar or even identical individual ground truth cut curves may exist among such alike images.

*Projected cut curve* refers to a curve along which a cut has not been carried out, although in case a cut is to be carried out, the cut is to be carried out along the projected cut curve.

*Actual cut curve* refers to a curve along which a cut has been carried out.

*Surface* is preferably used to reference a physical surface of an object or a non-physical surface within or outside an object. A surface may be flat or curved. Accordingly, *surface* as used in e.g. *median surface* and *cut path* may refer to a physical surface or a not yet cut surface.

Preferred embodiments of the invention have be found to provide good cutting paths not only for half-carcasses that have been provided by a cut substantially along the median plane but also for half-carcasses which have been cut substantially offset relatively to the median plane. Here "good" typically refers to that small amounts such as essentially no amounts of valuable meat is cut away. In addition, preferred embodiments may be able to provide an acceptable cutting path for half-carcasses which have some kind of destroyed structure(s), such as one or more broken bones. Further, preferred embodiments have shown to have a large tolerance with regards to size of the half-carcass and providing cutting paths with little or even essentially no influence originating from the size of the half-carcass.

In some embodiments, an individual ground truth cut curve represents a curve along which curve a cut is projected to be carried out or has been carried out. In other embodiments, an individual ground truth cut curve represent a curve being offset relatively to a curve along which curve a cut is projected to be carried out or has been carried out. In the latter case, a transformation between the curve along which a cut is projected to be carried out or has been carried out is known typically by a transformation metric, symbolically written as

$$GTc = G^{-1}(\widetilde{GT}c)$$ where $\widetilde{GT}c$ is the offset ground truth cut curve and $G^{-1}$ is the inverse of a transformation metric $G(GTc)$ offsetting the ground truth curve being a curve along which a cut is to be carried out or has been carried out.

In embodiments using offsetting, the first trained neural network localises in a digital image an offset actual cut curve and the transformation metric is typically used to provide a non-offset actual cut curve. A transformation metric may be a vector function which displaces the actual cut curve, e.g. by a constant offset.

Offsetting of ground truth cut curve has inter alia the advantage that the ground truth can be associated with features in an image, which features for a non-offset ground truth cut curve may be visually unclear or even covered by e.g. debris resulting e.g. from providing the half carcass by a cutting process.

*Projected cut curve* refers to an imaginary cut curve added to a training image. A projected cut curve may be user defined.

*Executed cut curve* refers to a curve along which a half carcass has been cut to at least partly remove the spinal column.

*Actual cut curve (ACc)* refers to a curve in the median surface along which curve the half carcass is to be cut.

*Individual ground truth cut angle progression (GTCAp)* refers to a ground truth cut angle progression is established individually for each digital image. By this, the ground truth is valid for an individual image. However, if two or more images are highly alike, similar or even identical ground truths may exist among such alike images.

An *actual cut angle progression* ACAp refers to a progression of a cut angle, where the cut angle may be describe by a function $CAp = f(s)$ where $s$ is a position parameter e.g. along a cut curve. The angle is preferably defined relatively to pre-defined surface, such as the median surface. However, the angle may be defined relatively to an arbitrarily surface with a known orientation relatively to the half carcass for which the cut angle progression ACAp is to be used.

*Determine* as used in e.g. *determine in said specific digital image an actual cut curve* and in *determine an actual cut angle progression ACAp in a digital image of a half carcass* is used to denote the process of positioning in a digital image the object in question.

The labelling *first, second* and third, used in connection with e.g. *first trained network, second trained network* and *third trained network,* are used only as identifiers and do not represent any time at which the networks are provided and do not represent any importance, merit or ranking of the networks relatively to each other. The same typically applies for the first, second and third training set. In addition, the trained networks may each be a stand-alone network or labelling *first, second and third* network may refer to a trained network being trained to determine either an actual cut curve (ACc), an actual cut angle progression (ACAp) and/or a further actual cut curve (FACc).

Without being bound by theory, the inventors have hypothesized that by training a network based on ground truth cut curves, the trained network is trained to identify features in images, which features are associated with a ground truth cut curve. Accordingly, when localizing an actual cut curve in a digital image of a half carcass, the trained network identifies, if present, similar features and outputs a curve described e.g. by a plurality of co-ordinates.

BRIEF DESCRIPTION OF THE FIGURES

The present invention and in particular preferred embodiments thereof will now be described in more details with regard to the accompanying figures. The figures show ways of implementing the present invention and are not to be construed as being limiting to other possible embodiments falling within the scope of the attached claim set.

    Fig. 1 is a schematically cross sectional view of a carcass,

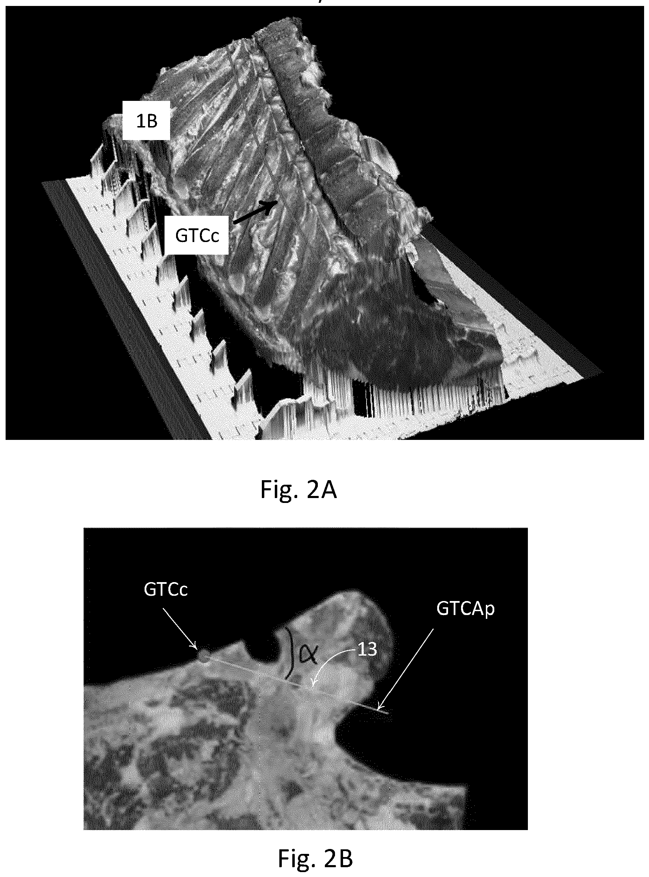

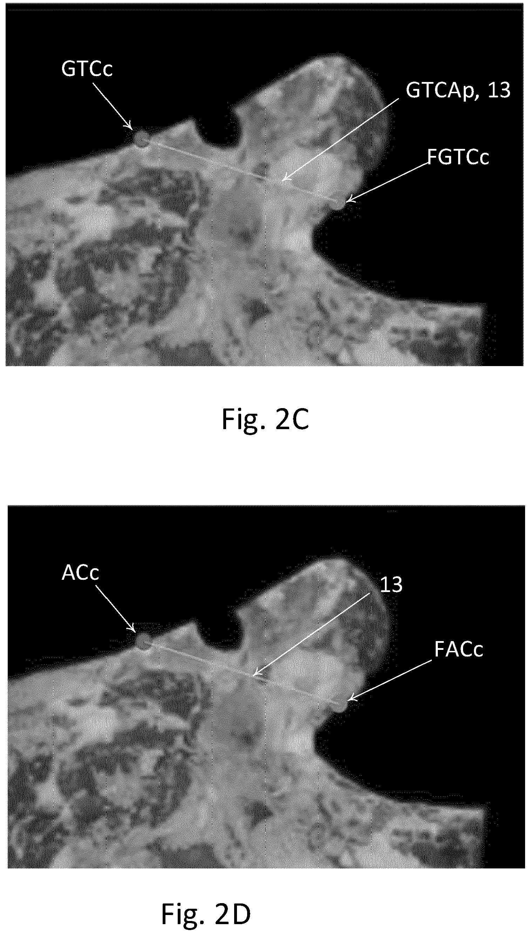

    Fig. 2A is an example of a digital image of a half carcass comprised in a first training set with a ground truth cut curve GTCc added to the image in accordance with a preferred embodiment, Fig. 2B illustrates in a cross sectional view an embodiment of a ground truth cut angle progression GTCAp. Fig. 2C illustrates in a cross sectional view another embodiment of a ground truth cut angle progression GTCAp in which the GTCAp is determined based on a further ground truth cut curve FGTCc. It is noted that in Fig. 2C the GTCAp is imaginary in the sense that the cut path 13 is provided as the path going through GTCc and FGTCc. Similarly, Fig. 2D illustrates that the trained neural network determines an actual cut curve ACc and a further actual cut curve FACc and the cut path is provided as the path going through ACc and FACc,

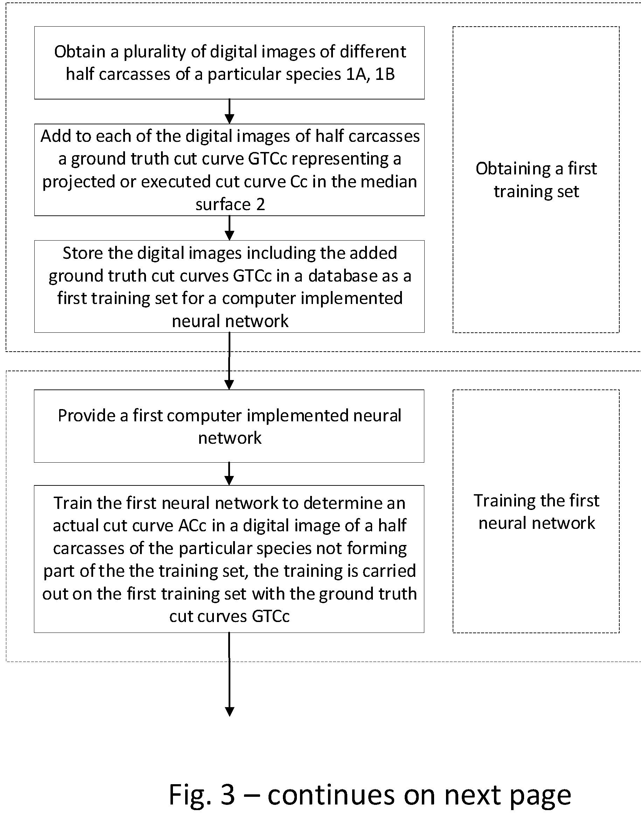

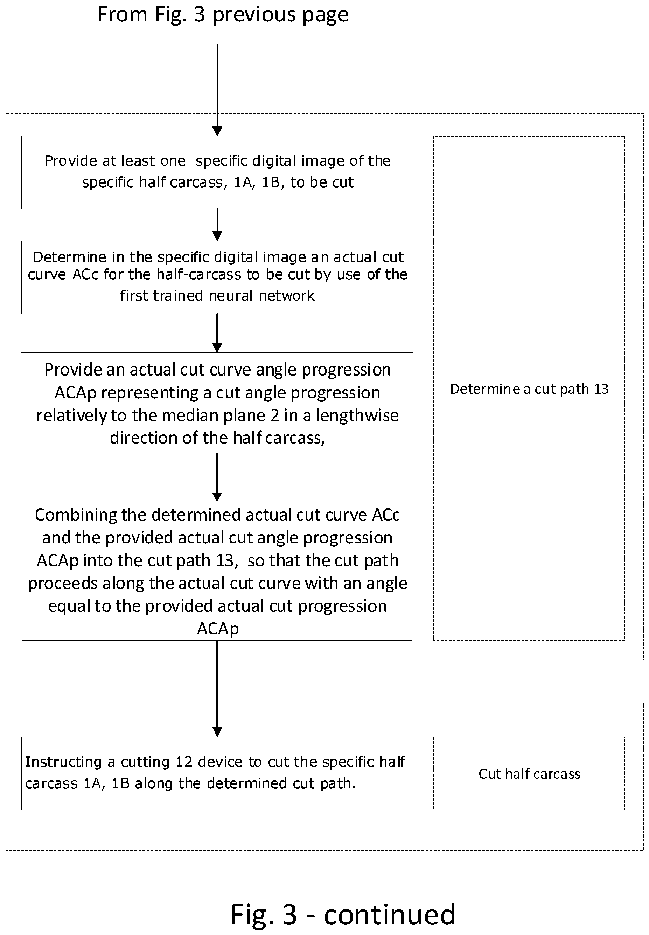

    Fig. 3 is a flow chart detailed a preferred embodiment of the method according to the invention,

    Fig. 4A is an example of a digital image of a half carcass for which a cut path is to be determined in accordance with a preferred embodiment.,

    Fig. 4B is an example of a determined actual cut curve in the image of Fig. 4A in accordance with a preferred embodiment,

    Fig. 4C is an example of a combination of the actual cut curve of Fig. 4B and a provided actual cut angle progression ACAp in a cut path in accordance with a preferred embodiment. The lines spanning the half-carcass (shown diagonally in Fig. 4C) illustrates the orientation of the surface of the median surface relatively to which the angle is provided (in the illustrated embodiment),

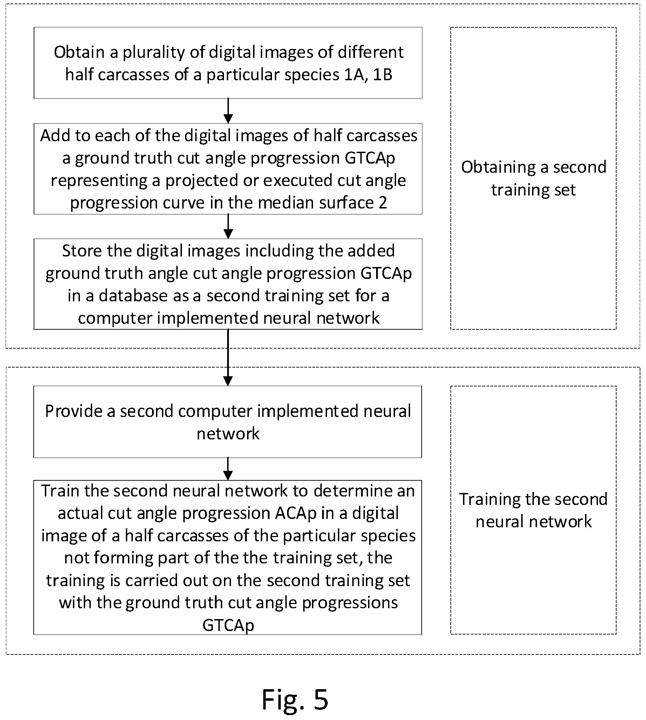

    Fig. 5 is a flow chart of an embodiment according to which a cut angle progression is determined by a neural network.

    Fig. 6 is an example on a mask applied to digital image of a training set, wherein the mask masks out the spinal canal.

DETAILED DESCRIPTION OF PREFERRED EMBODIMENTS

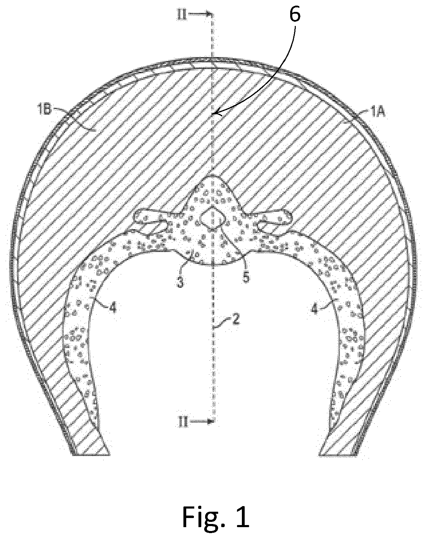

With reference to the accompanying figures, preferred embodiments will now be disclosed in details. As presented earlier, preferred embodiments relates to a computer implemented method of removing, at least partly, a spinal column from a specific half carcass 1A, 1B of a slaughtered animal of a particular species. In Fig. 1, a cross sectional view of a carcass is shown together with the median surface (a cut surface provide by cutting along line II-II of Fig. 1) along which the carcass is split to provide a half carcass. It is noted that the invention is not limited to operate only on a half carcass which has been provided by a split perfectly coinciding with the median surface, as preferred embodiment will be able to remove, at least partly, a spinal column from a half carcass being the result of a split offset from the median surface.

The wording "specific half carcass" is used to reference a carcass from which the spinal column is planned for removal. A specific half carcass does preferably not form part of a training for training a trained neural network as will be detailed below.

The specific half carcass 1A, 1B has been provided by a cut substantially along the median surface 2 of the slaughtered animal as illustrated in Fig. 1 by the line II-II. By this, a median cut surface 6 is provided on the specific half-carcass 1A, 1B. It is noted that the cut along the median surface 6 may be offset so that the depth of the spinal canal 5 is different for the two half carcasses and the cutting angle may also be titled relatively to what is disclosed in Fig. 1, since preferred embodiments of the invention may be capable of handling cuts being tilted and/or offset.

Preferred embodiments of the invention involves a neutral network to provide an actual cut curve ACc in a digital image of a half carcass along which actual cut curve ACc the half carcass is to be cut.

To obtain such a neural network, a first training set of images is provided. The labelling "first" is used to reference a first training set and a first neutral network for providing the actual cut curve ACc. Such a first training set preferably comprises a plurality of digital images of different half carcasses 1A, 1B of the particular species. The images of the half carcasses preferably comprise both right and left hand side half carcasses to mimic that both left and right hand side carcasses preferably are present when cutting is effectuated in a slaughter facility.

Each of the digital images of the first training set comprises an individual ground truth cut curve GTCc representing a projected or executed cut curve Cc in the median cut surface 6. A projected cut curve Cc refers to that a cut has not been carried out to remove the spinal canal 5 and an executed cut curve Cc refers to that a cut in the half carcass 1A, 1B has been carried out to remove the spinal canal 5. In both cases, the individual ground truth cut curve GTCc is added to the image. Preferably, such addition is carried out manually by a human operator, but may also be added automatically e.g. by use of a stencil or other automated processes. One example of a ground truth cut curve GTCc is illustrated in Fig. 2

In some embodiments, a trained neural network may further trained (or re-trained) based on the following. One or more digital images of a half carcass which has been cut in accordance with a preferred embodiment of the invention is/are obtained. A new ground truth cut curve GTCc is provided for the image(s) of the half carcass where the new GTCc is the cut path determined by the trained neural network and according to which the half carcass has been cut with the addition of an offset. The offset may be provided by an human operator and the offset represents where an improved cut could have been made in the half carcass. If the cut is found to be satisfactory, the offset may be set to zero. In case the cut has been to deep, the offset may be set proportional to the magnitude of the error represented by the depth. This is typically done for a number of cut half carcasses and the images with the new ground truth cut curve are used for re-training of the trained neural network.

Such an offset may be used for the first, the second and/or the third training set and thereby used to train one or more of the first, the second and/or the third neural network. Which of the network to re-train may depend on which of the network to improve. For instance, if the second neural network provides less optimal result, the second neural network is re-trained based on offsetting.

In embodiments, where the ground truth cut curve GTCc represent an executed cut curve Cc, the ground truth cut curve may be offset relatively to the executed cut curve Cc. Such offset may advantageous in situations where the executed cut curve Cc is provided by a cut being considered less than optimal whereby the offset curve can be used for training of the first trained network.

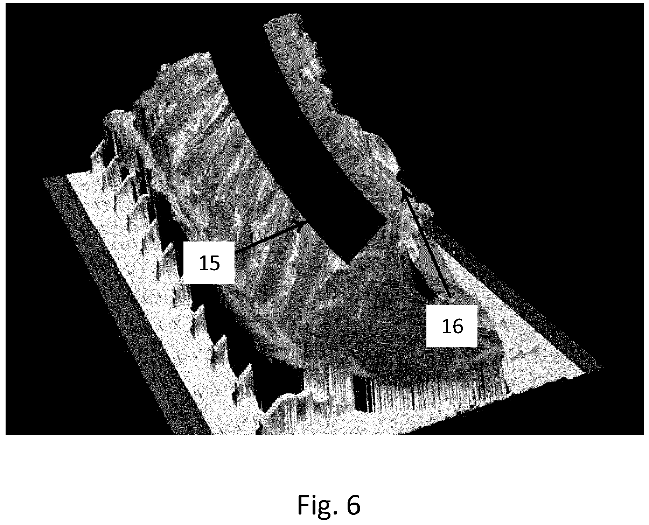

A ground truth cut curve can be seen as a reference curve according to which a cut is to be made and requires in general no prior knowledge of where the spinal canal 5 is located in the half carcass 1A, 1B. Accordingly, in preferred embodiments, the spinal canal 5 is masked out in images of the first training set, whereby training of the first neural network is forced to locate an actual cut curve ACc on the basis of other features in an image than the spinal canal 5. An example on masking is illustrated in Fig. 6, where the mask 15 is represented by a black area masking-out information about the half carcass below the mask 15.

After the first training set has been provided, training of a neural network is carried out on the first training set, which training provides a first trained neural network. The training may be carried out by a gradient descent optimization method.

Such a first trained neural network is computer implemented and training of the first trained neural network is carried out on the first training set with use of said ground truth cut curves GTCc. By being trained on images containing the ground truth cut curves, the first training neural network is trained to determine an actual cut curve ACc in a digital image of a half carcass 1A, 1B of the particular species, which digital image does not form part of the first training set. Or said in other words, the first trained neural network is trained to determine an actual cut curve ACc in an image not yet processed by the first trained neural network.

"Determine" refers in preferred embodiments to that the first trained neural network provides an actual cut curve for a particular image. Such an actual cut curve ACc is preferably a 3-dimensional curve expressed by a number of co-ordinates x, y and z in a co-ordinate system in which the half carcass 1A, 1B is located, that is an actual cut curve ACc is a 3-dimensional curve passing through a number of x,y,z co-ordinates. In preferred embodiments, the half carcass is arranged on a table or conveyer defining axis in the co-ordinate system.

With the first neural network trained, the first trained neural network is used to determine a cut path 13. Such a cut path is a path along which a cutting device, such as a saw is to cut in order to remove at least partly the spinal canal 5. It is noted that while a cut curve preferably may be understood as a 3-dimensional object, a cut path is preferably considered to be a 3 dimensional surface.

Determination of the cut path 13 comprising a number of steps and at one stage a specific digital image is provided of the specific half carcass 1A, 1B to be cut. With the digital image provided, the first training neural network determines in the specific digital image an actual cut curve ACc for the half-carcass to be cut.

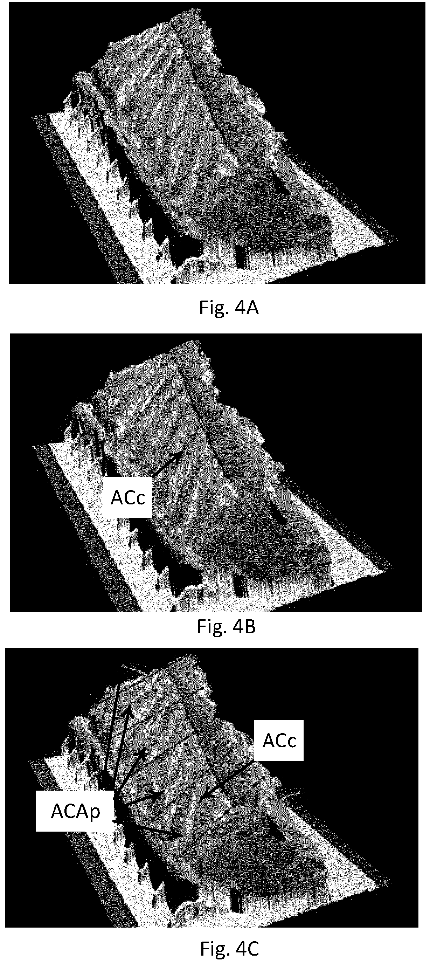

To provide a cut path 13, the determined actual curve ACc is in preferred embodiments combined with an actual cut angle progression ACAp representing a cut angle progression ACAp relatively to the median surface 2 in a lengthwise direction of the half carcass. An example of an actual cut angle progression ACAp is shown in Fig. 4c.

The concept of an actual cut angle progression ACAp, may for clarity reasons be written symbolically as:

$$\alpha_{ACAp} = F(s)$$

where $\alpha$ is an angle relatively to the median surface and $F$ ($s$) is a function providing $\alpha$ as function of a position parametersin a co-ordinate system in which the half carcass is located. It is noted, that while the above function provides an example on the actual cut angle progression ACAp, the invention is not limited to such or similar functional relationships.

As will be detailed in the following, an actual cut angle progression ACAp may be provided in different ways, and may even be a predetermined cut angle progression ACAp. It is furthermore noted, that the actual cut angle progression ACAp may be constant whereby the cut angle relatively to the median surface 2 is a fixed angle.

The determined actual cut curve ACc and the provided an actual cut angle progression ACAp is combined into the cut path 13. The combination is preferably carried out, so that said cut path 13 proceeds along the actual cut curve with an angle equal to the provided actual cut angle progression ACAp.

This combination is carried out on a computer which receives from the first trained neural network, the actual cut curve ACc and receives or holds the actual cut angle progression ACAp. The data representation of the cut path may be selected in accordance with a data representation to be given a cutting device, and may comprise a number of x,y,z co-ordinates and/or a vector representation of the cut path 13.

With the cut path provided, a computer controlled cutting device is instructed to cut the specific half carcass 1A, 1B longitudinally along the cut path.

To allow for the first neural network to be effectively trained to determine an actual cut curve ACc, the ground truth cut curves GTCc in one or more such as all of the digital images are represented by a data typically being a numerical representation of a curve or collection of points, such as coordinates in a coordinate system.

Such data is typically stored in separate file but may be embedded in a digital image file.

The ground truth cut curve(s) GTCc is/are in one more, such as all, of said plurality of digital images is/are user defined. However, vision software may be used either alone or in combination with user definitions to define ground truth cut curves GTCc.

While the ground truth cut curve GTCc may be provided for half carcasses not yet been cut to remove at least partly the spinal column, the ground truth cut curve (GTCc) in one or more, such as all, of the plurality of digital images may be a curve or points resembling a progression of an executed cut line in a half carcass (1A, 1B) of said particular species, where progression refers to the point of curve resembling an executed cut curve.

As detailed above, an actual cut angle progression ACAp may be provided in different ways. In preferred embodiments, the actual cut angle progression ACAp is provided by a trained neural network. In such embodiments, the method further provides a second training set, where the second training set comprises a plurality of digital images of different half carcasses 1A, 1B of the particular species. For each of the digital images an individual ground truth cut angle progression GTCAp is provided.

Based on the second training set, a second trained neural network is provided. Also the second trained neural network is computer implemented and trained to determine an actual cut angle progression ACAp in a digital image of a half carcass 1A, 1B of said particular species not forming part of said second training set, and training of the second trained neural network is carried out on said second training set with use of the ground truth cut angle progression GTCAp.

As for the training of the first trained neural network, the second trained network is trained to identify features in an image which features are associated with a curve, which for the second trained neural network is associated with first the ground truths and when deployed an actual cut angle progression ACAp.

Once trained, the second trained neural network is used to provide an actual cut angle progression ACAp by localizing said actual cut angle progression (ACAp) in a digital image.

It is noted that the digital image in which the first trained neural network determines an actual cut curve ACc is an image of the same half carcass for which the second trained neural network determines an actual cut angle progression ACAp. In some preferred embodiment, the digital image used by the first and the second trained neural network is the same digital image.

Further, the representation of the ground truth cut angle progression GTCAp may be similar to the representation of the ground truth cut curve GTCc, although a further dimension, namely the angle, preferably is contained in the representation of the ground truth cut angle progression GTCAp.

In preferred embodiments, the method uses a third trained neural network to determine a further actual cut curve FACc. Such a further actual cut curve FACc may be combined with an actual cut curve ACc determined by the first trained neural network into a cut path as a path going through the actual cut curve ACc and the further actual cut curve FACc.

In such preferred embodiments, the method comprises providing a third training set, wherein the third training set comprises a plurality of digital images of different half carcasses 1A, 1B of the particular species, wherein each of the digital images comprising a further ground truth cut curve FGTCc representing a further projected or executed cut curve FCc.

The third trained neural network is, as the first and the second computer implemented, and is trained based on the third training set to determine a further actual cut curve FACc in a digital image of a half carcass 1A, 1B of a particular species not forming part of the third training set. That is, preferably the training of the third trained neural network is carried out on the third training set with use of the further ground truth cut curve FGTCc.

Once the third neural network has been trained, it may be used to determine a further actual cut curve FACc. Such a determination may comprise providing at least one specific digital image of the specific half carcass 1B, 1B to be cut, and determine in the specific digital image the further actual cut curve FACc for said half carcass by use of the third trained neural network.

With the further actual cut curve FACc determined, it may be combined with the actual cut curve ACc determined by the first trained neural network into a cut path.

With the cut path determined, the cutting device may be instructed to cut the specific half carcass longitudinally along the cut path determined.

It may be generally preferred, that during the training of the first and/or the second neural network, the network is/are prevented from identifying specific features in the digital images including the spinal canal. To accomplish this, the area reproducing or likely reproducing the spinal canal can be hidden by a mask 15 positioned relative to a curve 16 (see Fig. 6) limiting the median cut surface near the top of the back, or said in another manner, the area reproducing or likely reproducing the spinal canal is preferably said to be blanked out in the images. Such a masking may be applied to the images of the first, the second and/or the third training set.

By hiding the spinal canal, the training of the neural networks forces the neural network to identify other features in the images than features associated with the spinal column. Without being bound by theory, the inventor has hypothesized that a more precise localization of an actual cut curve ACc and/or actual cut angle progression ACAp is obtainable as the number of identifiable features are larger that compared to if the spinal canal was used.

In a slaughter facility, half carcasses may be provided by an intended cut or what may be characterised as imprecise cut, where imprecise cuts may result in that part or sections of the half carcass is not present. To increase the neural networks' diversity to operate on different cuts including imprecise cuts at least some of the images of the fist training set and/or, at least some of the images of the second training set and/or at least some of the image of the third training set may only reproduce one or more sections of said half carcass. By this, the trained neural network may be able to determine actual cut curve ACc and/or actual cut angle progression ACAp in images where sections of the half carcass is not present or blurred.

Another common issue appearing in a slaughter facility is that the half carcass contained residues or other unintended matter. To increase the neural networks' diversity to operate on images containing such residues, at least some of the different half carcasses for which a digital image is comprised in the first training set and/or, at least some of images of the second training set and/or at least some of the images of the third training set may comprise residues, such as leftovers, or faults from providing the different half carcasses.

As disclosed above, it may be advantageous to blank out a section in the image comprising the spinal canal. In addition to this, or as an alternative, at least some of the digital images of the first training set and/or, at least some of the images of the second training set and/or at least some of the images of the third training set have been masked to blank out one or more features or sections of the half carcass. Such blank out could blank out the spinal canal but other features or regions which have shown to decrease the accuracy of the first and/or the second trained neural to determine an actual cut curve ACc and/or actual cut angle progression ACAp. Such features or regions could be blurred regions in the digital images, regions having features similar to those to be associated with the actual cut curve ACc and/or actual cut angle progression ACAp. Blanking out may also be used to enable the trained network to faster localization.

Although the first and the second training sets are disclosed as being separate training set, the first training set and the second training set may be the same training set. In such embodiments, a single 3-dimensional image may be used in the training set and the ground truths for the first training and the second training are associated with single 3-dimensional image. Instead of using a single 3-dimensional image, more images such as 2-dimensional or 3-dimensional images may be used.

Accordingly, and in preferred embodiments, digital images of the first training set and the digital images of the second training set are the same digital images.

It envisaged, that the invention is not limited to a particular animal as long as the animal has a spinal canal and can be split into half carcasses. However, invention is found to be particular useful where the slaughtered animal is a cow, a sheep, a pork, a cattle, a horse, a wild boar or a bison.

In preferred embodiments, the images of the training sets and the specific digital image are provided by a 3D camera, such as a stereo camera or a CT scanner. In preferred embodiments, a camera is used which operates based on triangulation, according to which the camera angle is oblique relatively to a laser line while the half carcass passes under the laser.

While the above description has been focussed towards method aspects of the invention, the invention also relates to a system for removing, at least partly, a spinal column from a specific carcass of a slaughter animal.

Preferred embodiments of the system typically comprises a cutting device configured to cut the specific carcass 1A, 1B longitudinally along a provided cut path. Such a cutting device may be a band saw or a circular saw. The cutting device typically comprises a guiding system which can orientate the cutting device so as to cut a half carcass longitudinally along a provided cut path. The guiding system is typically numerically controlled.

The system for removing also comprises computer system being configured to carry one or more preferred embodiments of the method so as to provide the cut path. The computer system is also configured to instruct the cutting device to cut along said provided cut path which in preferred embodiments involves that the computer system send instructions guiding system to orientate the cutting device according to the provided cut path.

The invention can be implemented by means of hardware, software, firmware or any combination of these. The invention or some of the features thereof can also be implemented as software running on one or more data processors and/or digital signal processors.

LIST OF REFERENCE SYMBOLS USED:

| 1A, 1B | Half carcass |
| 2 | Median surface |
| 3 | Spinal column |
| 4 | Rib |
| 5 | Spinal canal |
| 6 | Median cut surface |
| 13 | Cut path |
| 14 | Area |
| 15 | Mask |
| 16 | Curve (limiting the median cut surface near the top of the back) |
| $\alpha$ | Cut angle |
| Cc | Cut curve |
| FCc | Further projected or executed cut curve |
| ACc | Actual cut curve |
| FACc | Further actual cut angle curve |
| CAp | Cut angle progression |
| ACAp | Actual cut angle progression |
| GTCc | Ground truth cut curve |

| FGTCc | Further ground truth cut curve |
| GTCAp | Ground truth cut angle progression |

## Claims

1. A computer implemented method of removing, at least partly, a spinal column from a specific half carcass (1A, 1B) of a slaughtered animal of a particular species, said specific half carcass (1A, 1B) has been provided by a cut substantially along the median surface (2) of said slaughtered animal thereby providing a median cut surface (6) of said specific half-carcass (1A, 1B), the method comprising

   • provide a first training set comprising

      ○ a plurality of digital images of different half carcasses (1A, 1B) of said particular species, wherein each of said digital images comprising an individual ground truth cut curve (GTCc) representing a projected or executed cut curve (Cc), preferably located in said median cut surface (6);

   • provide a first trained neural network, said first trained neural network being computer implemented and trained to determine an actual cut curve (ACc) in a digital image of a half carcass (1A, 1B) of said particular species not forming part of said first training set, wherein training of said first trained neural network is carried out on said first training set with use of said ground truth cut curves (GTCc),
   • determining a cut path (13) by

      ○ provide at least one, such as one or more, specific digital image of said specific half carcass (1A, 1B) to be cut,
      ○ determine in said specific digital image(s) an actual cut curve (ACc) for said half-carcass to be cut by use of the first trained neural network,
      ○ provide an actual cut angle progression (ACAp) representing a cut angle progression relatively to a pre-defined surface, such as the median surface (2), in a lengthwise direction of the half carcass, or a further actual cut curve (FACc),
      ○ combining said actual cut curve (ACc) and said actual cut angle progression (ACAp) into said cut path (13), so that said cut path proceeds along said actual cut curve with an angle equal to said provided actual cut angle progression (ACAp), or combining said actual cut curve (ACc) and said further actual cut curve (FACc) into said cut path as a path as the path going through said actual cut curve (ACc) and said further actual cut curve (FACc),
• instructing a cutting device to cut the specific half carcass (1A, 1B) longitudinally along said provided cut path.

2. A method according to claim 1, wherein each of said ground truth cut curves (GTCc) are represented a by numerical representation of a curve or collection of points, such as coordinates in a coordinate system of the digital images.

3. A method according to claim 1 or 2, wherein said ground truth cut curve(s) (GTCc) in one more, such as all, of said plurality of digital images is/are user defined.

4. A method according to any of the preceding claims, wherein said ground truth cut curve (GTCc) in one or more, such as all, of said plurality of digital images is/are a curve or points resembling a progression of an executed cut line in a half carcass (1A, 1B) of said particular species.

5. A method according to any one of the preceding claims, wherein the method further comprising

• provide a second training set comprising

  ○ a plurality of digital images of different half carcasses (1A, 1B) of said particular species, wherein each of said digital images comprising an individual ground truth cut angle progression (GTCAp) representing a projected or executed cut angle progression (CAp) relatively to a pre-defined surface, such as the median surface;

• provide a second trained neural network, said second trained neural network being computer implemented and trained to determine an actual cut angle progression (ACAp) in a digital image of a half carcass (1A, 1B) of said particular species not forming part of said second training set, wherein training of said second trained neural network is carried out on said second training set with use of said ground truth cut angle progression (GTCAp), and

wherein said actual cut angle progression (ACAp) is provided by said trained second neural network determines said actual cut angle progression (ACAp) in said digital image.

6. A method according to any one of the preceding claims, wherein the method further comprising

• provide a third training set comprising

  ○ a plurality of digital images of different half carcasses (1A, 1B) of said particular species, wherein each of said digital images comprising a further ground truth cut curve (FGTCc) representing a further projected or executed cut curve (FCc);

• provide a third trained neural network, said third trained neural network being computer implemented and trained to determine a further actual cut curve (FACc) in a digital image of a half carcass (1A, 1B) of said particular species not forming part of said third training set, wherein training of said third trained neural network is carried out on said third training set with use of said further ground truth cut curve (FGTCc); and
• providing at least one specific digital image of said specific half carcass (1B, 1B) to be cut,
• determine in said specific digital image said further actual cut curve (FACc) for said half carcass by use of said third trained neural network.

7. A method according to any one of the preceding claims, wherein an area reproducing or likely reproducing the spinal canal is hidden in one or more of the digital images of the first and/or, when dependent on claim 5, of the second training set, and/or when depending on claim 6, of the third training set, by a mask (15) positioned relative to a curve limiting the median cut surface near the top of the back.

8. A method according to any one of the preceding claims, wherein at least some of the images of the fist training set and/or, when dependant on claim 5, at least some of the images of the second training set and/or when depending on claim 6, at least some of the images of the third training set only reproduce one or more sections of said half carcass.

9. A method according to any one of the preceding claims, wherein at least some of the different half carcasses for which a digital image is comprised in the first training set and/or, when dependant on claim 5, at least some of the images of the second training set, and/or when depending on claim 6, at least some of the images of the third training set comprising residues, such as leftovers, or faults from providing said different half carcasses.

10. A method according to any one of the preceding claims, wherein at least some of said digital images of the first training set and/or, when dependant on claim 5, at least some of the images of the second training set, and/or when depending on claim 6, at least some of the images of the third training set have been masked to black out one or more features or sections of the half carcass, such as the spinal canal.

**11.** A method according to any one of the preceding claims, wherein said digital images of the first training set and, when dependant on claim 5, said digital images of the second training set and when dependent on claim 6, said digital images of the third training set are the same digital images.

**12.** A method according to any one of the preceding claims, wherein the slaughtered animal is a cow, a sheep, a pork, a cattle, a horse, a wild boar or a bison.

**13.** A method according to any one of the preceding claims, wherein said digital images of the training sets and said specific digital image are provided by a 3D camera, such as a stereo camera, a CT scanner or a scanner using triangulation of laser light lines.

**14.** A system for removing, at least partly, a spinal column from a specific carcass of a slaughter animal, said system comprising

  • a cutting device configured to cut the specific carcass (1A, 1B) longitudinally along a provided cut path;
  • a computer system being configured

    ∘ to carry the method according to any one of the preceding claims to provide said cut path
    ∘ instruct the cutting device to cut along said provided cut path.

Fig. 1

Fig. 2A

Fig. 2B

Fig. 2C

Fig. 2D

Obtain a plurality of digital images of different
half carcasses of a particular species 1A, 1B

Add to each of the digital images of half carcasses
a ground truth cut curve GTCc representing a
projected or executed cut curve Cc in the median
surface 2

Store the digital images including the added
ground truth cut curves GTCc in a database as a
first training set for a computer implemented
neural network

Obtaining a first
training set

Provide a first computer implemented neural
network

Train the first neural network to determine an
actual cut curve ACc in a digital image of a half
carcasses of the particular species not forming
part of the the training set, the training is carried
out on the first training set with the ground truth
cut curves GTCc

Training the first
neural network

# Fig. 3 – continues on next page

From Fig. 3 previous page

Provide at least one specific digital image of the specific half carcass, 1A, 1B, to be cut

Determine in the specific digital image an actual cut curve ACc for the half-carcass to be cut by use of the first trained neural network

Provide an actual cut curve angle progression ACAp representing a cut angle progression relatively to the median plane 2 in a lengthwise direction of the half carcass,

Determine a cut path 13

Combining the determined actual cut curve ACc and the provided actual cut angle progression ACAp into the cut path 13, so that the cut path proceeds along the actual cut curve with an angle equal to the provided actual cut progression ACAp

Instructing a cutting 12 device to cut the specific half carcass 1A, 1B along the determined cut path.

Cut half carcass

Fig. 3 - continued

Fig. 4A

Fig. 4B

Fig. 4C

Obtain a plurality of digital images of different half carcasses of a particular species 1A, 1B

↓

Add to each of the digital images of half carcasses a ground truth cut angle progression GTCAp representing a projected or executed cut angle progression curve in the median surface 2

↓

Store the digital images including the added ground truth angle cut angle progression GTCAp in a database as a second training set for a computer implemented neural network

Obtaining a second training set

↓

Provide a second computer implemented neural network

↓

Train the second neural network to determine an actual cut angle progression ACAp in a digital image of a half carcasses of the particular species not forming part of the the training set, the training is carried out on the second training set with the ground truth cut angle progressions GTCAp

Training the second neural network

Fig. 5

Fig. 6

Europäisches
Patentamt

European
Patent Office

Office européen
des brevets

# EUROPEAN SEARCH REPORT

**Application Number**

EP 23 20 4842

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | US 8 915 773 B2 (NIELSEN ERIK VIND [DK]; HUMBOLDT BV [NL]) 23 December 2014 (2014-12-23) * abstract; claims 1-13; figures 1-8 * * column 1, line 4 – column 7, line 58 * | 1-14 | INV. A22B5/00 A22B5/20 G01N33/12 G06T7/00 G06V10/00 |
| A | EP 3 307 073 B1 (IHFOOD AS [DK]; SFK LEBLANC AS [DK]) 3 April 2019 (2019-04-03) * abstract; claims 1-16; figures 1-8 * * paragraphs [0001] – [0063] * | 1-14 | |
| A | WO 2023/063215 A1 (MAEKAWA SEISAKUSHO KK [JP]) 20 April 2023 (2023-04-20) * abstract; claims 1-12; figures 1-9 * * paragraphs [0001] – [0075] * & EP 4 316 246 A1 (MAEKAWA SEISAKUSHO KK [JP]) 7 February 2024 (2024-02-07) * abstract; claims 1-12; figures 1-9 * * paragraphs [0001] – [0105] * | 1-14 | |
| A | US 2007/275648 A1 (SATO ATSUSHI [JP]) 29 November 2007 (2007-11-29) * abstract; claims 1-5; figures 1-11C * * paragraphs [0001] – [0066] * | 1-14 | **TECHNICAL FIELDS SEARCHED (IPC)** A22B G06T G01N |
| A | WO 2020/120702 A1 (MAREL SALMON AS [DK]) 18 June 2020 (2020-06-18) * abstract; claims 1-21; figures 1a-14 * * page 1, line 3 – page 13, line 16 * | 1-14 | G06V A22C |

The present search report has been drawn up for all claims

| Place of search | Date of completion of the search | Examiner |
|---|---|---|
| The Hague | 6 March 2024 | Rojo Galindo, Ángel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 20 4842**

This annex lists the patent family members relating to the patent documents cited in  the above-mentioned European search report.
The members are as contained in the European Patent Office EDP file on
The European Patent Office is in no way liable for these particulars which are merely given for the purpose of information.

**06-03-2024**

| Patent document cited in search report | | Publication date | Patent family member(s) | | Publication date |
|---|---|---|---|---|---|
| US 8915773 | B2 | 23-12-2014 | DK | 2566339 T3 | 24-04-2017 |
| | | | EP | 2566339 A1 | 13-03-2013 |
| | | | ES | 2621460 T3 | 04-07-2017 |
| | | | PL | 2566339 T3 | 31-07-2017 |
| | | | US | 2013059513 A1 | 07-03-2013 |
| | | | WO | 2011138461 A1 | 10-11-2011 |
| EP 3307073 | B1 | 03-04-2019 | AU | 2016275699 A1 | 18-01-2018 |
| | | | DK | 178447 B1 | 29-02-2016 |
| | | | EP | 3307073 A1 | 18-04-2018 |
| | | | ES | 2729072 T3 | 30-10-2019 |
| | | | US | 9198441 B1 | 01-12-2015 |
| | | | WO | 2016198646 A1 | 15-12-2016 |
| WO 2023063215 | A1 | 20-04-2023 | EP | 4316246 A1 | 07-02-2024 |
| | | | JP | 2023058319 A | 25-04-2023 |
| | | | WO | 2023063215 A1 | 20-04-2023 |
| US 2007275648 | A1 | 29-11-2007 | AU | 2005232481 A1 | 27-10-2005 |
| | | | BR | PI0509868 A | 16-10-2007 |
| | | | CA | 2552307 A1 | 27-10-2005 |
| | | | CN | 1921767 A | 28-02-2007 |
| | | | EP | 1736057 A1 | 27-12-2006 |
| | | | JP | 4126027 B2 | 30-07-2008 |
| | | | JP | 2005304314 A | 04-11-2005 |
| | | | US | 2007275648 A1 | 29-11-2007 |
| | | | WO | 2005099462 A1 | 27-10-2005 |
| WO 2020120702 | A1 | 18-06-2020 | CA | 3122898 A1 | 18-06-2020 |
| | | | EP | 3893652 A1 | 20-10-2021 |
| | | | US | 2022026259 A1 | 27-01-2022 |
| | | | WO | 2020120702 A1 | 18-06-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8915773 B